# EUROPEAN PATENT APPLICATION

(11) **EP 3 936 176 A1**
(43) Date of publication of application: **12.01.2022**
(21) Application number: 20185085.6
(22) Date of filing: 09.07.2020
(51) Int. Cl.: A61M 15/08, A61M 15/00, A61M 16/10, A61M 16/00, A61N 1/44, H05H 1/00

(54) **SYSTEM AND PLASMA FOR TREATING AND/OR PREVENTING A VIRAL, BACTERIAL AND/OR FUNGAL INFECTION**

(71) Applicant: terraplasma GmbH, 85748 Garching (DE); Terraplasma medical GmbH, 85748 Garching (DE)
(72) Inventor: KIRSCH, Jens, 85241 Hebertshausen (DE); WACHTER, Hannes, 81249 München (DE); ROSKOPF, Claudia, 81249 München (DE); ZIMMERMANN, Julia, 81925 München (DE); MORFILL, Gregor, 81925 München (DE)
(74) Representative: Vossius & Partner Patentanwälte Rechtsanwälte mbB

(57) **Abstract**

A system for treating and/or preventing a viral, bacterial and/or fungal infection in the oral cavity and/or along the respiratory tract, in particular the interior of the nose, throat, trachea and/or lungs, of a patient by reactive species generated by plasma as well as a plasma for such use is disclosed. The system comprises a plasma source generating reactive species in a gas, the plasma source being configured to be located outside a body of the patient, and a species directing member forming at least one duct for guiding at least a part of the reactive species generated by the plasma source into the oral cavity and/or the respiratory tract.

## Description

### FIELD OF INVENTION

The invention relates to a system for treating and/or preventing viral, bacterial and/or fungal infection in the oral cavity and/or along the respiratory tract, in particular in the interior of the nose, throat, trachea and/or lungs, of a patient by reactive species generated by plasma. Furthermore, the invention relates to a method for plasma treatment of a patient as well as to plasma as obtainable by the system of the invention.

### BACKGROUND OF THE INVENTION

In 2020, a pandemic caused by a novel coronavirus, SARS-CoV-2, causing a respiratory syndrome (COVID-19) has become a global challenge of overwhelming importance. Despite worldwide intensive efforts, the treatment methods currently available are limited. There is therefore an urgent need for improved treatment options.

More generally, viral epidemics or pandemics occur repeatedly and pose a major threat to human life, health and economy. Examples for such pandemics or epidemics are the Spanish flu during 1918 to 1920, the severe acute respiratory syndrome (Sars) in 2002 and the Middle Eastern respiratory syndrome (Mers) in 2012 to 2016. Sars and Mers were both caused by coronaviruses. Sars had a death rate of more than 10% and spread to 37 countries in 2002. The Spanish flu is assumed to have been the deadliest pandemic in history, killing up to 100 million people between 1918 and 1920.

In the last two decades there has hardly been a phase in which no pandemic has occurred for a long time. As globalization progresses, it is assumed that not much will change in this development - rather, the problem will grow. With every new viral epidemic or pandemic, the problem is that vaccines are not ready quickly enough, so that a rapid increase in the number of people affected by the disease cannot be stopped for the time being. A slowdown in growth can be achieved by appropriate "social distancing", quarantine and hygiene measures. However, such measurements only can help to reduce the risk that health care systems become overloaded by particularly aggressive viruses.

Thus, there is an ongoing need for new treatment options of infections.

WO 2019/238863 A1 - incorporate herein by reference in its entirety - relates to a plasma device for the treatment of body surfaces, with a hand-held base body on which a plasma source is arranged, which is arranged to generate a non-thermal plasma, and with a spacer which is arranged, in order, in the mounted state, to define a distance between the plasma source and a body surface to be treated. The spacer can be detachably connected to the base body and/or to the plasma source and the plasma source can be detachably connected to the base body.

WO 2017/013211 A1 - incorporate herein by reference in its entirety - relates to an electrode assembly for generating a non-thermal plasma, comprising a first electrode and a second electrode which are electrically insulated from each other by means of a dielectric element and which are arranged at a distance from each other. The first electrode has a thickness of at least 10 µm when seen in the direction of the distance between the electrodes, and the second electrode has a thickness of at least 1 µm to maximally 5 µm or a thickness of at least 5 µm to maximally 30 µm when seen in the direction of the distance between the electrodes. The dielectric element has a thickness of at least 10 µm to maximally 250 µm.

### SUMMARY

According to a first aspect, the application discloses a system for treating and/or preventing a viral, bacterial and/or fungal infection in the oral cavity and/or along the respiratory tract, in particular the interior of the nose, throat, trachea and/or lungs, of a patient by reactive species generated by plasma. The system comprises a plasma source and a species directing member. The plasma source is configured to be located outside a body of the patient. The plasma source generates reactive species in a gas. The species directing member forms at least one duct for guiding at least a part of the reactive species generated by the plasma source into the oral cavity and/or the respiratory tract.

The system allows, for example, for the treatment of viral infections affecting the oral cavity and/or the respiratory tract, such as coronavirus (Sars-CoV-1, Mers, Sars-CoV-2), influenza, or adenovirus.

The reactive species generated by the plasma have the potential to inactivate viruses, bacteria and/or fungi. Thus, at least a part of the reactive species generated by the plasma source can be guided by the species directing member into the oral cavity and/or the respiratory tract of a patient having an infection where they can function to inactivate the viruses, bacteria and/or fungi. A patient having an infection can thus be treated by the system in order to inactivate the respective virus, bacteria and/or fungi.

The duct may comprise a first end through which at least part of the reactive species generated by the plasma source enter the duct and a second, open end through which at least part of said reactive species are released. Preferably, the first end is configured to be located outside the patient's body and/or the second end is configured to be located in the oral cavity and/or in the respiratory tract. For example, the second end can be configured to be located in the nose and/or in the oral cavity, such as between 1 mm and 30 mm in the nasal cavity and/or between 1 mm and 10 cm in the oral cavity.

The duct may be at least partly formed by an elongate tubular member. The tubular member may be flexible or rigid. The tubular member may have an arcuate shape. For example, the tubular member may be a Guedel Tubus.

The tubular member may have a length of at least 1 cm, at least 2 cm, at least 3 cm, at least 5 cm, at least 10 cm, or at least 30 cm. The tubular member may have a length of less than 30 cm or less than 10 cm. Such tubular members have shown to be particularly effective for delivering the reactive species to the location to be treated. Such lengths may be particularly useful, for example, when the plasma source is mounted on a hand held device or component. The plasma source of the present invention could be provided in a hand held device as described by applicants in WO 2019/238863 A1, which is incorporated herein by reference in its entirety.

The length of the tubular member, however, may also be between 10 cm and 500 cm, between 10 cm and 400 cm, or between 20 cm and 200 cm. It has surprisingly been found by the inventors that even if the tubular member is quite long, e.g. more than 50 cm or more than 100 cm, at least a part of the reactive species can be effectively guided from the plasma source where they are generated to the end of the tubular member wherefrom they are released, e.g. into the oral cavity or the respiratory tract, and can still provide for an effective treatment. Without wanting to be bound by theory, it is assumed that such lengths may be particularly helpful when treating more distal regions of the respiratory tract, such as regions below the trachea (e.g. for treating the lungs and/or the bronchi).

Depending on the region to which the species are being delivered, the tubular member may have an inner diameter of at least 0.2 cm, at least 0.5 cm, at least 1 cm, at least 1.5 cm, or at least 2 cm. This may be the diameter at the second, open end of the tubular member. Without wanting to be bound by theory, it is assumed that the reactive species may still be delivered with such diameters to provide for an effective treatment. If necessary, a gas flow through the tubular member may be caused to enhance transport of the species through the tubular member, as explained in more detail below.

The tubular member preferably has a diameter of less than 3 cm, less than 2 cm or less than 1,5 cm, for example at the second end. Such diameters may be appropriate for introducing the tubular member into the nose and/or into the mouth of the patient.

The tubular member may be made from a polymeric material. Preferably the material comprises silicone and/or polyvinyl chloride. More preferably the tubular member may consist of silicone and/or polyvinyl chloride.

The plasma source preferably is configured to generate a non-thermal plasma and/or a cold atmospheric plasma. Such a plasma is particularly suited for generating reactive species having the potential of inactivating viruses, bacteria and/or fungi. Moreover, depending on the Cold Plasma design, it is possible to reduce the risk of creating reactive species that could be harmful to the patient.

The plasma source may comprise a first electrode, a second electrode and a dielectric member, wherein at least a part of the dielectric member is disposed between the first electrode and the second electrode. The plasma source may be configured, for example, as described in WO 2017/013211 A1 or WO 2019/238863 A1, which are incorporated by reference herein in their entirety.

The second electrode may be structured such that it comprises a plurality of edges configured to cause surface micro discharges upon application of an electrical voltage to the electrodes, particularly in the form of an alternating voltage. The edges may be formed via protruding structural elements and/or via recessed portions of the electrode and/or via one or more openings provided in the electrode. Such configuration may provide for a more effective and/or uniform plasma generation at the second electrode.

The second electrode may thus be a structured electrode, in particular a structured ground electrode.

Such structured electrode may comprise one or more segments that may be straight, curved and/or spirally shaped.

For example, the electrode may comprise a first segment forming a base from which a plurality of second segments which are electrically coupled to the base extend. Said second segments could also be referred to as branches while the first segment could be referred to as a base. The second segments may extend with or without contacting each other. For example, the second segments could be parallel, such as parallel straight lines or parallel serpentines (e.g., sinuous shaped). The first segment may be straight. The electrode could also be referred to as a comb shaped electrode in the context of the present disclosure.

Alternatively, the second electrode could be provided with a plurality of straight or curved segments that cross and/or overlap each other at a plurality of points (with or without being electrically contacted at the crossing points). Such structure may be referred to as a mesh shaped electrode in the context of the present disclosure.

Alternatively, the second electrode may have or comprise a spiral structure, for example in the form of a circular spiral or an angular spiral, or a meandering structure.

If the second electrode has one or more first and/or second segments, for example if it is formed as a comb, mesh or spiral, the segments preferably have a width of at least 50 µm to at most 200 µm. Without wanting to be bound by theory, it is believed that such width is favorable for providing a compact electrode design that achieves a sufficient number of surface discharges. The width preferably is measured in a plane in which the electrode extends. The width preferably is perpendicular to the electrode's thickness.

The first and second segments could be integrally formed.

The spiral, mesh or comb may be essentially planar.

The second electrode may be made by a printing process.

The second electrode may be embedded in the dielectric member. Such embedding, in particular in connection with the above-mentioned printing process and/or the above-mentioned electrode shapes, is believed to allow an efficient and cost-effective fabrication of the second electrode in a compact manner.

Preferably, the first electrode may have an essentially planar shape. In particular, the first electrode may be a laminar shaped high-voltage electrode. In other words, the first electrode may be provided as an essentially continuous sheet of conductive material, in particular a planar sheet. This allows for a cost effective manufacture. However, also a structured electrode may be used as the first electrode, if desired.

When the first electrode and the second electrode are essentially planar, the first electrode may extend in a first plane and the second electrode may extend in a second plane that is essentially parallel to said first plane.

The first electrode and/or the second electrode may be exposed to gas, in particular to gas in the species directing member and/or in a chamber (see below). Preferably, only the second electrode is exposed to the gas in the species directing member and/or in the chamber. The plasma generating the reactive species preferably occurs at the exposed electrode, in particular at the second electrode.

The system may further comprise an energy source for energizing the plasma source and a control unit configured to control a power supply from the energy source to the plasma source. The energy source may comprise a battery (e.g. a rechargeable battery) and/or an external power supply (e.g. for connecting the device to the grid).

The control unit may be configured to apply an alternating current and/or an alternating voltage to the first electrode and/or the second electrode. The alternating voltage may have an amplitude of at least 0.5 kVpp. The alternating voltage may have an amplitude of 3 kVpp or less. For example, the alternating voltage may have an amplitude between 0.5 kVpp and 3 kVpp.

Alternatively or additionally, the alternating voltage may have a frequency of at least 2 kHz. The alternating voltage may have a frequency of less than 90 kHz. For example, the alternating voltage may have a frequency of between 2 kHz and 90 kHz.

In particular, the control unit may be configured to apply an alternating current and/or an alternating voltage to the first electrode while the second electrode may be a ground electrode.

Preferably, the control unit is configured to adjust the voltage applied to the first electrode. Adjustment of the voltage allows for an adjustment of the reactive species generated by the plasma. For example, adjustment of the voltage allows to influence the oxygen chemistry and/or the nitrogen chemistry of the plasma. In particular, such voltage adjustments may be used to influence the amount of oxygen and/or nitrogen species in the plasma, such as the amount of O₃, N₂O, N₂O₅, HNO₂, HNO₃, and H₂O₂, see Y. Sakiyama et al., J. Phys. D: Appl. Phys. 45 (2012). Without wanting to be bound by theory, it is believed by the present inventors that certain viruses may be more susceptible to deactivation in the presence of certain reactive species. For example, it is believed that Sars-CoV-2 (and, likely, other coronaviruses) are more susceptible to deactivation in the presence of nitrogen species. The effectiveness of the device for inactivating certain viruses can thus be increased by adjusting the plasma composition. For the treatment of coronavirus, including Sars-CoV-2, it is currently envisaged to adjust the plasma composition such that a density of at least 1 ppm or at least 2 ppm are achieved for one or more of the following reactive species: HNO₂, HNO₃, and/or H₂O₂.

The plasma source may form a duct through which the gas flows, wherein a first electrode of the plasma source at least partially surrounds said duct. The first electrode may form part of the duct or even constitute the duct. As such, the first electrode may be substantially cylindrical. A second electrode of the plasma source may be at least partially or completely arranged in the duct, for example, along a main axis of the duct. The second electrode may be a structured electrode. For example, the first electrode may be formed as a structured electrode having a generally cylindrical shape. The second electrode may comprise apertures and/or protruding portions. The second electrode may be flexible. Such configuration allows to provide the plasma source in a space and cost efficient manner, with low maintenance costs, e.g. as part of a duct through which a flow of gas is provided to the patient.

The system may further comprise a chamber in which the reactive species are created and from which the reactive species are directed to the patient. Such chamber may be disposed between the plasma source and the species directing member. The chamber preferably comprises a chamber outlet opening that is fluidly connected to the first end of the species directing member. A cross section of the chamber may be larger than a cross section of the chamber outlet opening and/or a cross section of the species directing member along a longitudinal axis thereof.

The plasma source may form a surface region delimiting at least part of the chamber, so that the reactive species generated by the plasma source are released into the chamber. At least a portion of the surface region may be substantially planar. When the plasma source itself forms a duct or chamber, at least a portion of the surface region may be a tubular segment or circumferential.

The first electrode and/or the second electrode may be exposed to gas in the chamber. Only the first electrode or only the second electrode may be exposed to gas in the chamber, preferably only the second electrode.

The chamber outlet opening may be opposite the surface region formed by the plasma source with respect to the interior of the chamber. The duct of the species directing member may extend from the chamber in a direction substantially perpendicular, oblique or parallel to the surface region. The duct of the species directing member may extend from the chamber in a direction substantially perpendicular, oblique or parallel to the substantially planar portion of the surface region.

The chamber may have a volume of at least 3 cm³, at least 5 cm³, or at least 10 cm³. The chamber may have a volume of 100 cm³ or less, 50 cm³ or less, or 30 cm³ or less. Such dimensions have been shown to provide for sufficient volume for generating the required reactive species while still ensuring a compact design of the device (e.g., as a hand held device).

The chamber may be formed in a chamber member. The chamber formed in the chamber member may have at least one open side surface. This open side surface may at least partially be closed by the plasma source when the chamber member and the plasma source are attached to each other.

The chamber member may be detachable from the plasma source and/or removably connectable to the plasma source. The chamber member may be manually detachable and/or manually connectable to the plasma source. The chamber member may be detachable from the plasma source and/or removably connectable to the plasma source via a snap-fit and/or a friction-fit connection.

The species directing member may be integrally formed with the chamber member. Alternatively, the structure forming the chamber could be permanently or releasably coupled to the species directing member.

The chamber member and/or the species directing member may comprise a radio-frequency identification tag. Such tag may help to ensure that a particular chamber member and/or a particular species directing member is only used once, or is only used repeatedly with a particular patient.

The chamber member may comprise a gas inlet allowing a gas flow into the chamber. The gas flowing into the chamber may be ambient air. However, the gas flowing into the chamber may also be provided with a predetermined composition (i.e. distinct from ambient air) from a gas source. Using such gas with a predetermined composition instead of ambient air allows to influence the generated reactive species, for example whether more reactive oxygen species or more reactive nitrogen species are generated.

The system may further comprise a gas flow generator configured to generate a gas flow through the species directing member and/or a gas flow into the chamber. Such gas flow generator may be a fan, a source of pressurized gas and/or a medical ventilator.

The gas flow generator (in particular when configured as a fan) may be positioned at least partially within the chamber or adjacent to the chamber. Alternatively or additionally, the gas flow generator (in particular when configured as a fan) may be positioned in the species directing member. For example, the gas flow generator may be positioned at the chamber outlet opening and/or at the first end of the species directing member.

Alternatively, the gas flow generator may be positioned outside the chamber, for example when configured as a fan, a pressurized gas source or a medical ventilator. In particular, such gas flow generator may be positioned upstream of the chamber.

In either case, the gas inlet may be configured to direct the gas flow into the chamber in a direction towards the plasma source. In particular, the gas inlet may be configured to direct the gas flow into the chamber in a direction towards the surface region formed by the plasma source. For example, the gas inlet may be configured to direct at least part or most of the gas flow into the chamber in a direction substantially perpendicular, oblique or parallel to the substantially planar portion of the surface region formed by the plasma source.

The gas inlet may be located along a wall of the chamber that is opposite the surface region formed by the plasma source with respect to the interior of the chamber. Alternatively or additionally, a gas inlet could be located along a lateral wall of the chamber, e.g., a lateral wall that is substantially perpendicular or oblique to the surface region formed by the plasma source.

The gas inlet may be located adjacent the chamber outlet opening.

The system may further comprise a gas source, in particular a source of pressurized gas. Said source of pressurized gas may be used to provide gas having a predetermined composition. In addition, such source may be used to force the gas to flow through the system.

The pressure of the pressurized gas may be at least 200 kPa. The pressure of the pressurized gas may be 1000 kPa or less. Without wanting to be bound by theory, such pressures have been found to provide an adequate flow of gas through the system. As the skilled person will appreciate, the pressure may have to be adapted in view of, e.g., the cross section of the gas inlet, the chamber, the gas outlet, and/or the duct.

The pressurized gas may be air or O₂, or a mixture of O₂ and N₂, or a mixture with a noble gas (e.g., Ar or He). Such mixture may be, for example, a mixture of O₂, N₂ and a noble gas. The noble gas may be, for example, Ar and/or He.

The gas in the gas source may comprise at least 40 %wt of oxygen, at least 50 %wt of oxygen, at least 70 %wt of oxygen, or at least 90 %wt of oxygen. Oxygen is routinely supplied to patients suffering from certain respiratory disorders (e.g., Sars-CoV-2). When using such composition, the gas which is anyways supplied to the patient could be enriched with reactive species, for example in certain intervals.

Alternatively, the gas in the gas source may comprise at least 80 %wt of nitrogen, at least 85 %wt of nitrogen, at least 90 %wt of nitrogen, or at least 95 %wt of nitrogen. By using such gas compositions, the amount of reactive species containing nitrogen may be increased. As indicated above, it is believed that certain viruses, bacteria or fungi may be more easily inactivated by reactive species containing nitrogen.

When using a noble gas, the gas mixture may contain at least 80% or at least 90% of said noble gas (e.g., Ar or He). In particular, a medical grade argon gas mixture could be used. Such noble gas could be mixed with O₂ and/or N₂ in the system (e.g., from ambient air). The presence of a noble gas makes it easier to generate plasma.

The system may further comprise a connection tube conducting the gas from the gas source to the plasma source. The connection tube may conduct the gas from the gas source into the chamber through the gas inlet.

The system may further include a flow regulator. The flow regulator may be configured to provide for a gas volume flow from the gas source of at least 0.1 standard liter per minute or at least 0.3 standard liter per minute. Alternatively or additionally, the flow regulator may be configured to provide for a gas volume flow from the gas source of 1.0 standard liter per minute or less, or 0.8 standard liter per minute or less.

The system may be configured for at least partially inactivating one or more pathogens in air exhaled by the patient. For this purpose, the exhaled air may be led to a further plasma source of the system in order to at least partially inactivate one or more pathogens in the exhaled air. Alternatively or additionally, the system may further comprise a filter for filtering one or more pathogens from the exhaled air. The filter may absorb or adsorb at least part of the reactive species within the exhaled air. Such filter may be an air filter provided by a fibrous or porous material. When both, a further plasma source and an air filter are provided, the further plasma source may be located upstream of the filter. In this manner, reactive species generated by the further plasma source may inactivate pathogens caught in the filter.

The system may further comprise an exhalation tube for receiving exhaled air from the patient. The exhalation may be fluidly connected to the further plasma source and/or to the filter.

The system may be a hand-held device. In particular, the plasma source and a user interface (e.g., a button or a display) for controlling said plasma source may be provided as part of the hand-held device, which may comprise a battery. The chamber member and/or the species directing member could then be releasably attachable to said hand-held device. With such configuration, a single hand-held device could be used for treating multiple patients. The chamber member and/or the species directing member could then be replaced when switching from one patient to another.

The system may further comprise a mechanical ventilator, wherein the species directing member is at least partially formed by a ventilation tube for intubating the patient. Such mechanical ventilator may be a positive pressure ventilator. When using such mechanical ventilator, the plasma source may be located inside a housing of the mechanical ventilator or it may be provided as a separate component that is removably attachable to said ventilator.

The mechanical ventilator may comprise a bladder, wherein the reactive species generated by the plasma source are conducted into the bladder. In this case, the reactive species in the bladder will be supplied to the patient with the gas in the bladder when the bladder is compressed.

Alternatively or additionally, the reactive species may be mixed with gas supplied by the mechanical ventilator after the gas is pressurized by the ventilator (e.g., after the gas is ejected from the bladder). For example, the reactive species could be sucked into a flow of pressurized gas provided by the ventilator by means of the venturi effect. For example, the plasma source may be located outside a housing of the mechanical ventilator. The plasma source may then be connected to the ventilation tube via a Y connector.

The system may further comprise an expandable cuff configured to restrict airflow through the respiratory tract. The cuff may be configured to be expanded at a position distal from the second end of the species directing member. When desired, or required in view of the reactive species generated, such expandable cuff may be used to prevent the reactive species from reaching more distal portions of the respiratory tract (e.g., the lungs and/or the bronchi) and/or more distal portions of the digestive tract (such as the stomach). In this manner, the treatment can be restricted, for example, to the interior of the nose and/or the oral cavity.

The described system is particularly suited for treating and/or preventing a viral, bacterial and/or fungal infection of a patient in the oral cavity and/or respiratory tract, in particular the interior of the nose, throat, trachea and/or lungs.

The species directing member does not necessarily have to be configured to be inserted into the oral cavity and/or the nose of the patient. For example, the species directing member could be formed as a mask (e.g., a nasal mask, a mouth mask or a full-face mask).

As shown in the examples, it was observed that plasma, preferably the plasma as obtainable by the herein described system, is suited to treat a viral, bacterial and/or fungal infection. In the context of the present invention, the viral, bacterial and/or fungal infection is localized in the oral cavity and/or along the respiratory tract, in particular the interior of the nose, throat, trachea and/or lungs of the patient to be treated. Accordingly, the present invention relates to the use of the herein described system for plasma treatment of a viral, bacterial and/or fungal infection in the oral cavity and/or along the respiratory tract, in particular the interior of the nose, throat, trachea and/or lungs.

Further, the present invention relates to a method for plasma treatment of a viral/bacterial and/or fungal infection in a patient, wherein the method comprises the step of introducing the species directing member of the system described herein into the respiratory tract, in particular into the interior of the nose, the throat, trachea and/or lungs, and/or into the oral cavity.

In the context of the present invention, the infection(s) to be treated with the reactive oxygen species generated by plasma, preferably by plasma as obtainable by the herein described system, is (are) (a) viral, bacterial and/or fungal infection(s).

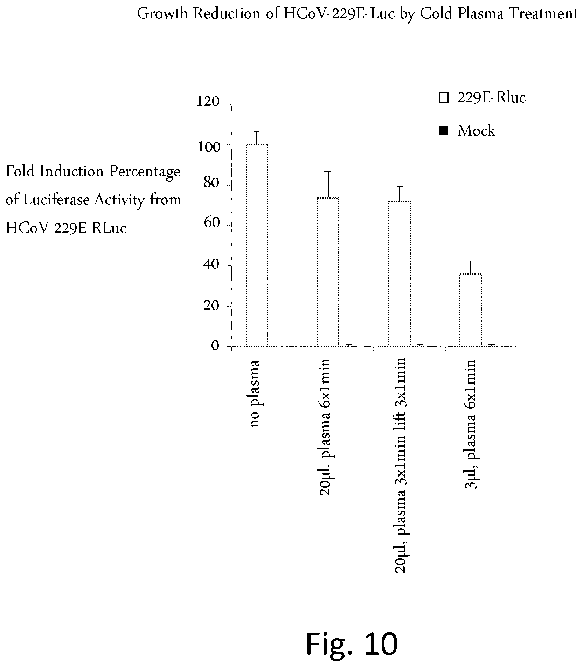

In the context of the present invention the viral infection to be treated is in particular a viral infectious disease selected from the group consisting of infections by coronaviruses, preferably severe acute respiratory syndrome coronavirus (SARS-CoV), SARS-CoV-2, Middle East respiratory syndrome coronavirus (MERS-CoV), influenza viruses, parainfluenza viruses, respiratory syncytial viruses (RSV), rhinoviruses, adenoviruses, noroviruses, enteroviruses, human metapneumoviruses, herpesviruses, preferably HSV-1, HSV-2, VZV, EBV, HCMV, HHV-6, HHV-7, HHV-8. Plasma for use in the treatment of a viral infectious disease caused by coronaviruses has not be reported. As shown in Fig. 10, the it was observed herein for the first time that plasma reduces the coronavirus infection. Accordingly, the present invention relates to plasma, preferably plasma as obtainable by the herein described system, for use in the treatment of a viral infectious disease mediated by coronaviruses, preferably SARS-CoV, SARS-CoV-2, or MERS-CoV.

Further, the bacterial infection to be treated in the context of the present invention is an infectious disease caused by bacterial infections mediated by gram positive or gram negative pathogens. It has been demonstrated that plasma can be used to reduce the growth of gram positive bacteria, such as *Staphylococcus aureus, Staphylococcus capitis, Methicillin-resistant Staphylococcus aureus, Deinococcus radiodurans, Staphylococcus epidermidis,* and *Enterococcus faecalis;* see, e.g., Zimmermann et al., New Journal of Physics 14 (2012), 073037. Further, the usability of plasma for the reduction of gram negative bacteria, such as *Escherichia coli* K12, *Escherichia coli, Escherichia coli* 0157:H7 and *Pseudomonas aeruginosa* has been reported; see, e.g., Maisch et al., Journal of Industrial Microbiology & Biotechnology 39, 9 (2012), 1367-75; Klämpfl et al., Applied Environ Microbiology 78, 15 (2012), 5077-5082; Maisch et al., PLoS One 7, 4 (2012), 34610; and Morfill et al., New Journal of Physics 11 (2009), 115019 10pp.

Further, the fungal infection to be treated in the context of the present invention is an infectious disease caused by fungi, or parasites.

### BRIEF DESCRIPTION OF THE DRAWINGS

The subject-matter of the application will be explained in more detail with reference to preferred exemplary examples which are illustrated in the attached drawing, in which
Fig. 1 is a schematic perspective view of a system according to a first example.
Fig. 2 is a schematic cross-sectional view of the system.
Fig. 3 is a schematic cross-sectional view of a system according to a second example.
Fig. 4 is a schematic perspective view of a system according to a third example.
Fig. 5 is a schematic view of a variation of the system illustrated in Fig. 4.
Figures 6a and 6b are schematic views of further examples.
Fig. 7 is a schematic view of a further example, comprising a bladder.
Figs. 8a and 8b illustrate alternative configurations of the plasma source of the system.
Figs. 9a to 9g illustrate several examples of electrodes.
Fig. 10 reports the coronavirus reduction after plasma treatment. The following experiments were conducted: (A) Controls: virus without plasma treatment (no plasma); (B) mock transfection with plasma treated medium (20µL or 3µL; in case the CAP secretes cytotoxic compounds into the medium); (C) The coronavirus HCoV-229E was genetically modified to encode renilla luciferase (229E-Rluc). The virus was suspended in DMEM + 5 % FCS. Caco-2 cells were infected with the treated virus samples. After 48 hours incubation, the cells were lysed and the luciferase activity in the supernatant was determined.

### DETAILED DESCRIPTION OF EXAMPLES

Examples according to the present application will be described with reference to the drawings in which identical or similar reference signs designate identical or similar elements. The features of the examples may be combined with each other, unless specifically noted otherwise.

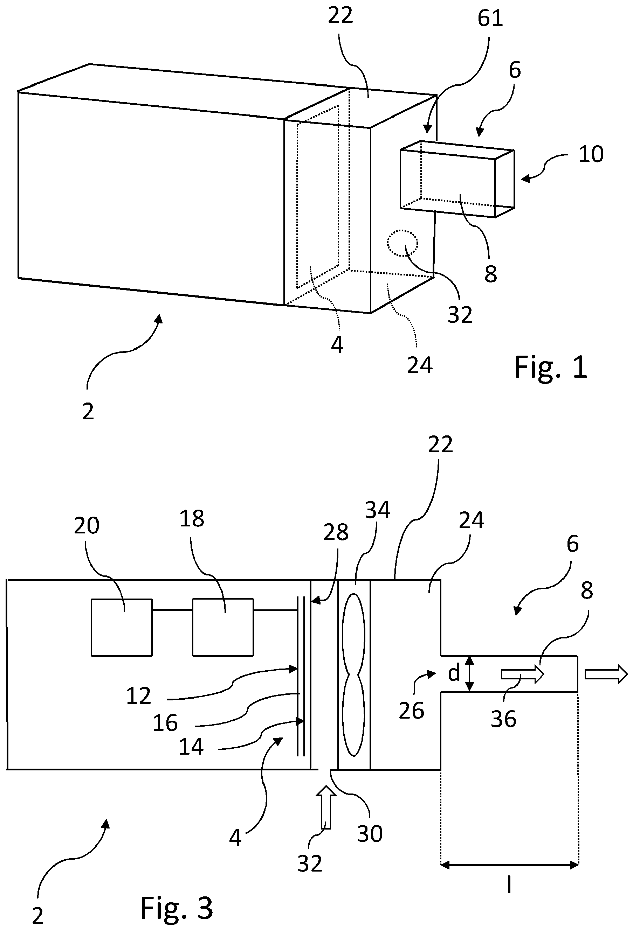

Fig. 1 shows a schematic view of a system 2 according to a first example. The system is configured for treating and/or preventing a viral, bacterial and/or fungal infection in the oral cavity and/or along the respiratory tract of a patient by reactive species generated by plasma. The system 2 comprises a plasma source 4 generating reactive species in a gas. The plasma source 4 is configured to be located outside the body of the patient.

The system further comprises a species directing member 6 forming at least one duct for guiding at least a part of the reactive species generated by the plasma source 4 into the oral cavity and/or the respiratory tract of the patient.

The duct comprises a first end 61 through which at least part of the reactive species generated by the plasma source 4 enter the duct and a second, open end 10 through which at least part of said reactive species are released. The first end 61 of the duct is configured to be located outside the patient's body. The second end 10 of the duct is configured to be located or to be arranged in the oral cavity and/or in the respiratory tract of the patient.

The duct is preferably at least partly formed by an elongate tubular member 8. The tubular member 8 may be flexible or rigid.

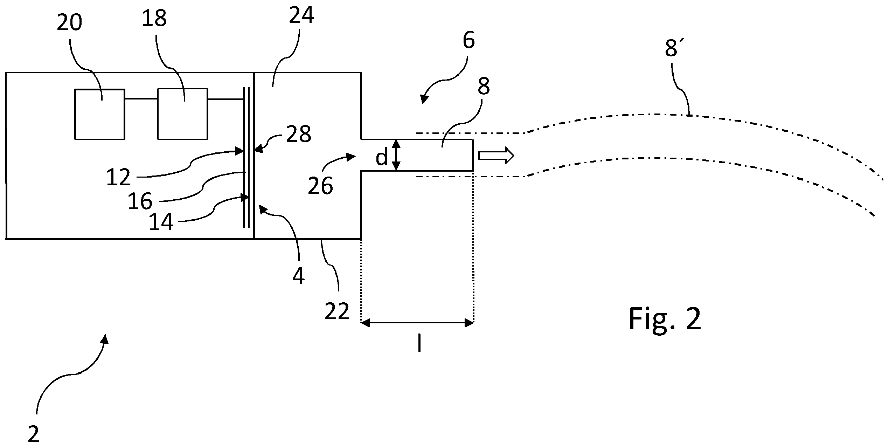

As illustrated in Fig. 2, the tubular member 8 may have a length 1 and/or an inner diameter d such that the tubular member 8 is suited to be inserted with its second end 10 into the oral cavity and/or a nasal cavity as part of the respiratory tract of the patient. For example, the length of the tubular member 8 may be between 0.5 cm and 10 cm. The tubular member 8 preferably has an inner diameter d of at least 0.2 cm, more preferably at least 0.5 cm.

The tubular member 8 is preferably made from a polymeric material, for example from a polymeric material comprising silicone and/or polyvinyl chloride. More preferably, the tubular member 8 consists of silicone and/or polyvinyl chloride.

The plasma source 4 is configured to generate a non-thermal plasma and/or a cold atmospheric plasma and comprises - as schematically illustrated in Fig. 2 - a first electrode 12, a second electrode 14 and a dielectric member 16, wherein at least a part of the dielectric member 16 is disposed between the first electrode 12 and the second electrode 14.

The first electrode 12 is preferably a laminar shaped high-voltage electrode and the second electrode 14 is preferably a structured ground electrode. Preferably, the structure of the second electrode 14 is configured such that the second electrode 14 is suited to generate surface micro discharges for generating the plasma when a voltage is applied to the first electrode 12 and/or the second electrode 14, here preferably to the first electrode 12. The second electrode 14 may be embedded in the dielectric member 16.

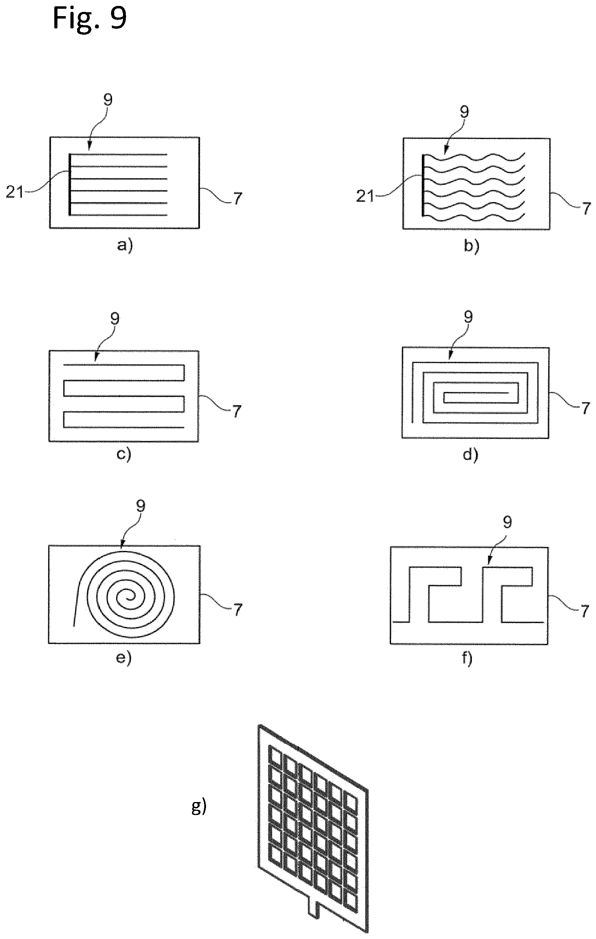

Figures 9a to 9g illustrate examples of a electrodes. Figures 9a to 9f show plan views showing a first electrode - here indicated by reference sign 7 - and a second, structured electrode - here indicated by reference sign 9. The electrodes 7, 9 are separated from each other by a dielectric member (not shown in Figures 9a to 9f), as described above. As shown in Fig. 9a, the second electrode 9 may comprise a first segment 21 forming a base from which a plurality of second segments or branches extend. The second segments are electrically coupled to the first segment 21. As illustrated, the first segment 21 may have a linear structure. As shown in Fig. 9a, the second segments may extend parallel, for example as straight lines. Thus, the electrode 9 may be "comb-shaped". Alternatively, as shown in Fig. 9b, the second segments e.g. may be shaped wavelike or sinusoidally.

Fig. 9c shows an example of a second electrode 9 having a serpentine shape. Fig. 9e shows an example of an electrode 9 which is spiral shaped, Fig. 9d shows an electrode 9 having the shape of a rectangular spiral. Fig. 9f shows an example of an electrode 7 having a meandering shape.

Fig. 9g shows - in a perspective view - an electrode having a mesh-shaped structure.

The first electrode 12 and/or the second electrode 14 may have an electrode surface between 1 cm² and 100 cm². The electrode surface may be rectangular, for example sized between 1 cm times 1 cm and 10 cm times 10 cm. Different sizes of the electrode surface allow for individual adjustments. For example, a smaller size of the electrode surface may be suited for a child, while a larger size is preferred for a grown-up person.

The system further comprises an energy source 18 for energizing the plasma source 4 and a control unit 20 configured to control a power supply from the energy source 18 to the plasma source 4. The control unit 20 is preferably configured to adjust a value of a voltage applied to the first electrode 12. Adjusting the voltage allows for an adjustment of the relation between the oxygen-chemistry and the nitrogen chemistry of the plasma, as explained above.

The system further comprises a chamber 24 disposed between the plasma source 4 and the species directing member 6. The chamber 24 comprises a chamber outlet opening 26. A first end of the species directing member 6, which may be identical to the first end 61 of the duct, is connected to the chamber outlet opening 26. A cross section of the chamber 24 is preferably larger than a cross section of the chamber outlet opening 26 and/or a cross section of the species directing member 6 along a longitudinal axis thereof. In this manner, the plasma source 4 may be provided with a relatively large surface area. This allows to generate a large amount of reactive species in the chamber 24 in a relatively short time.

The plasma source 4 preferably forms a surface region 28 delimiting at least part of the chamber 24, wherein the reactive species generated by the plasma source 4 are released into the chamber 24. According to this example, at least a portion of the surface region 28 is substantially planar.

Only the second electrode 14 is preferably exposed to gas in the chamber 24.

The chamber outlet opening 26 is located, for example, opposite the surface region 28 formed by the plasma source 4 with respect to the interior of the chamber 24. The duct of the species directing member 6 extends from the chamber 24 in a direction substantially perpendicular to the surface region 28. The chamber 24 may have a volume between 3 cm³ and 100 cm³.

The chamber 24 is formed in a chamber member 22. Preferably, the chamber member 22 is manually detachable from the plasma source 4 and/or removably connectable to the plasma source 4, for example via a snap-fit and/or a friction-fit connection.

According to a variation, the species directing member 6 may further form a second duct for guiding at least a part of the reactive species generated by the plasma source 4 into the oral cavity and/or the respiratory tract of the patient. Thus, the first mentioned duct may, for example, be used for a treatment of the oral cavity, whereas the other duct may, for example simultaneously, be used for a treatment of the nasal cavity.

As indicated in Fig. 2 by a dash-dotted line, the tubular member may comprise an arcuate shape 8'. The length of the tubular member 8 with such arcuate shape 8' may range, for example, from 3 cm to 10 cm. For example, the tubular member 8 and/or its arcuate shape 8' may be in the form of a Guedel Tubus.

More generally, the tubular 8 may have a length in the order of 1 m or 2 m. As surprisingly found by the inventors, even such a long tubular member is suited to effectively guide at least a part of the reactive species generated by the plasma source 4 to the patient, in particular when an adequate flow is provided therethrough.

The chamber 24 may comprise one or more gas inlets 32 through which gas flows into the chamber, e.g., ambient air.

Fig. 3 shows a second example which might be regarded as a variation of the first example. If not otherwise described in the following, the second example can be designed as the first example.

The system according to the second example comprises a gas flow generator 34, for example in the form of a fan, configured to generate a gas flow 36 through the species directing member 6 to the patient and/or a gas flow into the chamber 24. This is advantageous because the gas flow 36 provides for an effective transportation of the reactive species through the elongate member 8 to the patient.

Preferably, the system further comprises one or more gas inlets 30 which allow for a gas flow 32 into the chamber 24. As shown in Fig. 3, the one or more inlets 30 may be arranged upstream of the flow generator 34 whereas the outlet 26 of the chamber 24 may be arranged downstream of the flow generator 34. Alternatively, the gas flow could be generated at one or more of the one or more gas inlets 30 and/or after the outlet 26 (e.g., by arranging a fan in the tubular member 8)

As illustrated in Fig. 3, the gas flow generator 34 may be positioned at least partially within the chamber 24. Alternatively, the gas flow generator 34 may be positioned adjacent to the chamber 24 or in the species directing member 6. For example, the gas flow generator 34 may be positioned at the chamber outlet opening 26 and/or at the first end of the species directing member 6 or the first end 61 of the duct.

The gas inlet 30 may be configured to direct the gas flow 32 into the chamber 24 in a direction towards the plasma source 4, preferably in a direction towards the surface region 28 formed by the plasma source 4. In the illustrated example, the gas inlet 30 is configured to direct at least part or most of the gas flow 32 into the chamber 24 in a direction substantially parallel to the surface region 28 formed by the plasma source 4.

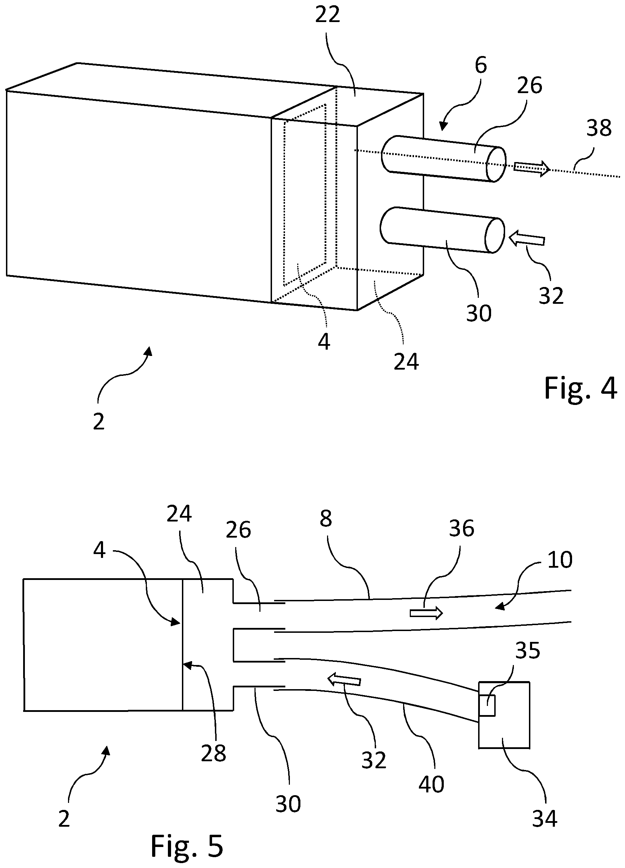

Fig. 4 shows a third example. If not otherwise described in the following, the third example can be designed as the first and/or second example.

The gas inlet 30 of the system according to the third example is configured to direct at least part or most of the gas flow 32 into the chamber 24 in a direction substantially perpendicular to the substantially planar portion of the surface region 28 formed by the plasma source 4.

As illustrated, the gas inlet 30 may be located opposite to the surface region 28 formed by the plasma source 4 with respect to the interior of the chamber 24. The gas inlet 30 may be in the form of a short tube. The gas may be ambient air.

The system may further comprise a gas source. For example, as illustrated in Fig. 5, the gas flow generator 34 which is here located outside the chamber 34 may comprise the gas source. The gas source is preferably a source of pressurized gas, where the pressure of the pressurized gas is at least 200 kPa and/or 1000 kPa or less.

The pressurized gas may be air, O₂, or a mixture of O₂ and N₂, or a mixture of O₂, N₂ and a noble gas, for example Ar and/or He. As indicated above, the oxygen content and the nitrogen content in the gas supplied to the plasma source 4 (e.g., in the pressurized gas) may be varied in order to achieve a desired composition of the reactive species generated by the plasma.

The system may further comprise a connection tube 40 conducting the gas from the gas source or the gas flow generator 34 to the plasma source 4 or to the chamber 24. Preferably, the connection tube 40 conducts the gas from the gas source into the chamber 24 through the gas inlet 30. In view of normal hospital settings, such connection tube may be, for example, at least 20 cm, at least 50 cm or at least 1 m long.

The system may further include a flow regulator 35, for example as a part of the flow generator 34, configured to provide for a gas volume flow of at least 0.1 standard liter per minute from the gas source.

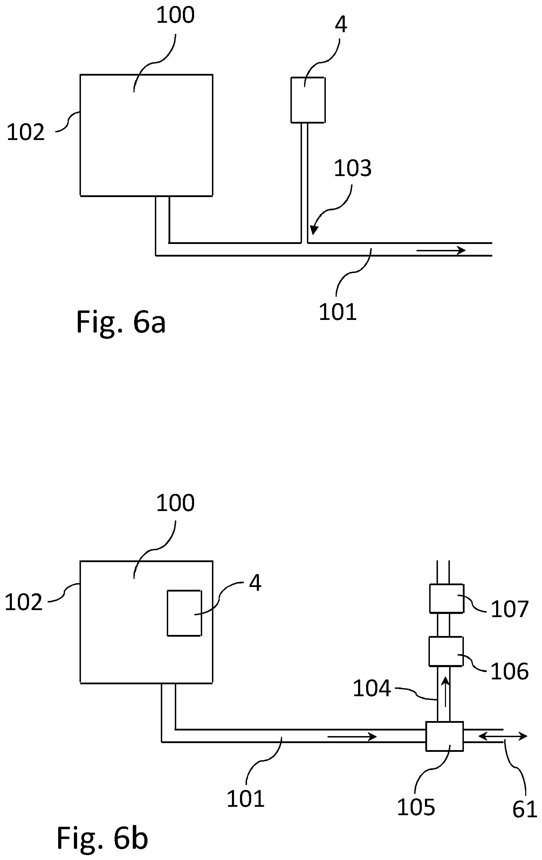

Figures 6a and 6b illustrate schematically further examples. The system may further comprise a mechanical ventilator 100, for example in the form of a positive pressure ventilator, where the species directing member is at least partially formed by a ventilation tube 101 for intubating the patient. As illustrated in Fig. 6a, the plasma source 4 may be located outside a housing 102 of the mechanical ventilator 100. Alternatively, as illustrated in Fig. 6b, the plasma source 4 may be located inside the housing 102 of the mechanical ventilator 100. If the plasma source 4 is located outside the housing 102 of the mechanical ventilator 100, the plasma source 4 may be connected to the ventilation tube 101 via a Y connector 103.

Alternatively, the system may comprise a mechanical ventilator 100, for example a positive pressure ventilator, and the species directing member may be formed as a nasal mask, a nose piece, or a full-face mask (not shown). In this case, the species directing member may, but does not necessarily have to be, configured to extend into the oral cavity and/or the respiratory tract. For example, the reactive species may be guided to the nasal mask, nose piece, or full-face mask (e.g., by being entrained in the pressurized gas from the ventilator or through a separate connection fluidly connected with the nasal mask, nose piece, or full-face mask) and inhaled by the patient when the patient takes a breadth.

As shown exemplarily in Fig. 6b, the system may further comprise an exhalation tube 104 for receiving exhaled air from the patient. The exhalation tube 104 may be connected to a valve member 105 designed to regulate the direction of the air exhaled by the patient. The valve member 105 may be further connected to the ventilation tube 101 and further designed to regulate the direction of the inhaled air.

As illustrated, the system may additionally comprise a further plasma source 106, connected to the exhalation tube 104 such that the exhaled air is at least partially led to the further plasma source 106. Thus, one or more pathogens can be inactivated at least partially by the further plasma source 106.

The system may further comprise a filter 107, preferably arranged downstream the further plasma source 106, for filtering one or more pathogens from the exhaled air. The filter 107 may be provided by a fibrous or porous material.

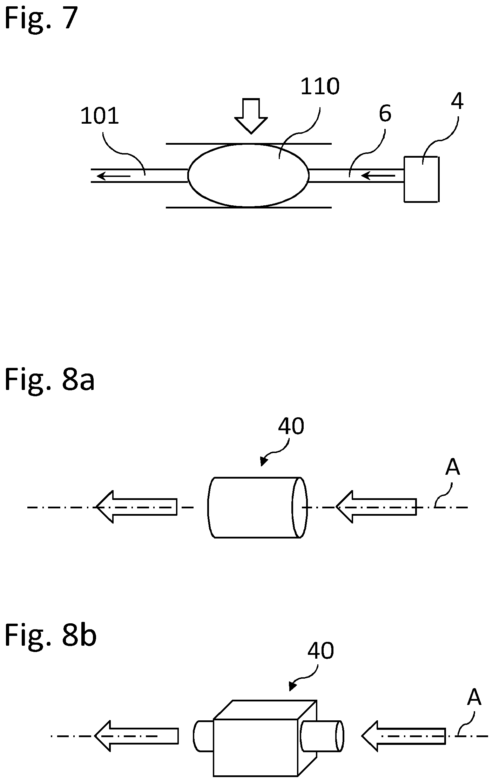

Fig. 7 shows an example where the mechanical ventilator comprises a bladder 110. Thus, the reactive species can be conducted from the plasma source 4 via the species directing member 6 into the bladder 110 and further from the bladder 110 via the ventilation tube 101 to the patient. As sketched in Fig. 7, the plasma source 4 may be arranged outside the bladder 110. Alternatively, the plasma source 4 may be arranged within the bladder 110.

Figures 8a and 8b illustrate a further alternate design of the plasma source. The plasma source may form a duct 40 through which the gas flows, wherein a first electrode of the plasma source is a part of the duct 40, constitutes the duct 40, and/or is arranged at least partially aroundthe duct 40. The duct 40 may be shaped cylindrically, for example - as sketched in Fig. 8a - having a circular cross section or - as sketched in Fig. 8b - having a rectangular cross section.

A second electrode of the plasma source may be arranged at least partly within the duct 40 along a main axis A of the duct 40, e.g. along a longitudinal symmetry axis of the cylinder. Here, preferably the second electrode is a structured electrode. The duct 40 may be directly coupled to the species directing member 6 such that the reactive species generated by the plasma source 4 are guided from the duct 40 - for example via or by a gas flow generator 34 - directly into the species directing member 6 and through the species directing member 6 to the patient.

Fig. 10 reports the coronavirus reduction after plasma treatment. The plasma device used in these tests is the one described in this application. The details of the experiment underlying the results shown in Fig. 10 are as follows: The human coronavirus 229E (HCoV-229E) is one of four coronaviruses responsible for the common cold. For the reporter assay, the virus HCoV-229E was genetically modified to encode renilla luciferase. Renilla luciferase is an enzyme that catalyzes the reaction of luciferin with oxygen and ATP to oxyluciferin, CO₂ and AMP. The reaction produces light. When the virus (HCoV-229E) is active and multiplies in infected cells, it also produces renilla luciferase. The light intensity in a sample mixed with the substrate luciferin correlates with the amount of virus. When a substance (here the cold plasma (CAP)) has a viricidal effect, the virus should not be able to replicate in infected cells, so less luciferase and less light is produced. In the experimental setup 3 µl or 20µL of a HCoV-229E virus solution (3x10^7 pfu/sample) were treated with CAP in a 96-well microtiter plate either for 6 minutes, or two times for 3 minutes. The experimental design was chosen to imitate the behavior of a coronavirus in an organic fluid such as saliva. The results of Fig. 10 report that a 6 minutes CAP treatment led to more than 60% reduction in luciferase activity. These test results indicate that coronaviruses can be inactivated in an organic fluid (e.g., in saliva).

While the invention has been described in detail in the drawings and forgoing description, such description is to be considered illustrative or exemplary and not restrictive. Variations to the disclosed examples can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

The invention relates, for example, to the following aspects:
1. A system for treating and/or preventing a viral, bacterial and/or fungal infection in the oral cavity and/or along the respiratory tract, in particular the interior of the nose, throat, trachea and/or lungs, of a patient by reactive species generated by plasma, the system comprising:
   a plasma source generating reactive species in a gas, the plasma source being configured to be located outside a body of the patient,
   a species directing member forming at least one duct for guiding at least a part of the reactive species generated by the plasma source into the oral cavity and/or the respiratory tract.
2. The system of aspect 1, wherein the duct comprises a first end through which at least part of the reactive species generated by the plasma source enter the duct and a second, open end through which at least part of said reactive species are released, preferably wherein the first end is configured to be located outside the patient's body and/or the second end is configured to be located in the oral cavity and/or in the respiratory tract.
3. The system of aspect 1 or 2, wherein the duct is at least partly formed by an elongate tubular member.
4. The system of any of the preceding aspects, wherein the tubular member has an arcuate shape.
5. The system of aspect 4, wherein the tubular member is a Guedel Tubus.
6. The system of any of aspects 3 to 5, wherein the tubular member is flexible or rigid.
7. The system of any of aspects 3 to 6,
   wherein the tubular member has a length of at least 1 cm, at least 2 cm, at least 3 cm, at least 5 cm, at least 10 cm, or at least 30 cm; and/or
   wherein the tubular member has a length of less than 30 cm or less than 10 cm.
8. The system of any of aspects 3 to 7, wherein the length of the tubular member is between 10 cm and 500 cm, between 10 cm and 400 cm, or between 20 cm and 200 cm.
9. The system of any of aspects 3 to 8, wherein the tubular member has an inner diameter of at least 0.2 cm, at least 0.5 cm, at least 1 cm, at least 1.5 cm, or at least 2 cm.
10. The system of any of aspects 3 to 9, wherein the tubular member is made from a polymeric material, preferably wherein the material comprises silicone and/or polyvinyl chloride, more preferably wherein the tubular member consists of silicone and/or polyvinyl chloride.
11. The system of any of the preceding aspects, wherein the plasma source generates a non-thermal plasma and/or a cold atmospheric plasma.
12. The system of any of the preceding aspects, wherein the plasma source comprises a first electrode, a second electrode and a dielectric member, wherein at least a part of the dielectric member is disposed between the first electrode and the second electrode.
13. The system of aspect 12, wherein the first electrode is a laminar shaped high-voltage electrode and/or wherein the second electrode is a structured ground electrode, for example a mesh or comb shaped electrode.
14. The system of aspect 12 or 13, wherein the second electrode is embedded in the dielectric member.
15. The system of aspect 12, 13 or 14, wherein the first electrode and/or the second electrode is exposed to gas in the species directing member, preferably wherein only the first electrode or only the second electrode is exposed to gas in the species directing member, in particular only the second electrode.
16. The system of any of the preceding aspects, further comprising an energy source for energizing the plasma source and a control unit configured to control a power supply from the energy source to the plasma source.
17. The system of aspect 16, wherein the control unit is configured to apply an alternating current and/or voltage to the first electrode.
18. The system of any of the preceding aspects, wherein the plasma source forms a duct through which the gas flows, wherein a first electrode of the plasma source is a part of the duct or constitutes the duct.
19. The system of aspect 18, wherein a second electrode of the plasma source is arranged along a main axis of the duct, wherein the second electrode is preferably at least partly arranged within the duct.
20. The system of aspect 18 or 19, wherein the second electrode is a structured electrode.
21. The system of any of the preceding aspects, further comprising a chamber disposed between the plasma source and the species directing member, wherein the chamber comprises a chamber outlet opening, and wherein a first end of the species directing member is connected to the chamber outlet opening, preferably wherein a cross section of the chamber is larger than a cross section of the chamber outlet opening and/or a cross section of the species directing member along a longitudinal axis thereof.
22. The system of aspect 21, wherein the plasma source forms a surface region delimiting at least part of the chamber, wherein the reactive species generated by the plasma source are released into the chamber, preferably wherein at least a portion of the surface region is substantially planar or a tubular segment or circumferential; and/or
   wherein the first electrode and/or the second electrode is exposed to gas in the chamber, preferably wherein only the first electrode or only the second electrode is exposed to gas in the chamber.
23. The system of aspect 22, wherein the chamber outlet opening is opposite the surface region formed by the plasma source with respect to the interior of the chamber; and/or
   wherein the duct of the species directing member extends from the chamber in a direction substantially perpendicular or oblique or parallel to the surface region, preferably in a direction substantially perpendicular or oblique or parallel to the substantially planar portion of the surface region.
24. The system of any of aspects 21 to 23, wherein the chamber has a volume of at least 3 cm³.
25. The system of any of aspects 21 to 24, wherein the chamber has a volume of at least 5 cm³.
26. The system of any of aspects 21 to 25, wherein the chamber has a volume of at least 10 cm³.
27. The system of any of aspects 21 to 26, wherein the chamber has a volume of 100 cm³ or less.
28. The system of any of aspects 21 to 27, wherein the chamber has a volume of 50 cm³ or less.
29. The system of any of aspects 21 to 28, wherein the chamber has a volume of 30 cm³ or less.
30. The system of any of aspects 21 to 29, wherein the chamber is formed in a chamber member, preferably wherein the chamber member is detachable from the plasma source and/or removably connectable to the plasma source, more preferably manually detachable and/or manually connectable, for example via a snap-fit and/or friction-fit connection.
31. The system of aspect 30, wherein the chamber member comprises a radio-frequency identification tag.
32. The system of any of aspects 21 to 31, wherein the chamber member comprises a gas inlet allowing a gas flow into the chamber.
33. The system of any of the preceding aspects, further comprising a gas flow generator configured to generate a gas flow through the species directing member and/or a gas flow into the chamber.
34. The system of aspect 33, wherein the gas flow generator is a fan, in particular a fan with an impeller.
35. The system of aspect 33 or 34, wherein the gas flow generator is positioned at least partially within the chamber or adjacent to the chamber; or
   wherein the gas flow generator is positioned in the species directing member.
36. The system of any of aspects 33 to 35, wherein the gas flow generator is positioned at the chamber outlet opening and/or at the first end of the species directing member.
37. The system of any of aspects 33 to 36, wherein the gas flow generator is positioned outside the chamber, preferably upstream of the chamber.
38. The system of any of aspects 32 to 37, wherein the gas inlet is configured to direct the gas flow into the chamber in a direction towards the plasma source, preferably in a direction towards the surface region formed by the plasma source.
39. The system of aspect 38, wherein the gas inlet is configured to direct at least part or most of the gas flow into the chamber in a direction substantially perpendicular or oblique or parallel to the substantially planar portion of the surface region formed by the plasma source.
40. The system of aspect 38 or 39, wherein the gas inlet is located opposite or lateral to the surface region formed by the plasma source with respect to the interior of the chamber.
41. The system of aspect 38, 39 or 40, wherein the gas inlet is located adjacent the chamber outlet opening.
42. The system of any of the preceding aspects, wherein the gas is ambient air.
43. The system of any of aspects 1 to 42, further comprising a gas source, preferably a source of pressurized gas.
44. The system of aspect 40, wherein the pressure of the pressurized gas is at least 200 kPa.
45. The system of aspect 40 or 41, wherein the pressure of the pressurized gas is 1000 kPa or less.
46. The system of aspect 43, 44 or 45, wherein the pressurized gas is air or O₂, or a mixture of O₂ and N₂, or a mixture of O₂, N₂ and Ar or a noble gas, for example He.
47. The system of any of aspects 40 to 43, wherein the gas in the gas source comprises at least 40 %wt of oxygen, preferably at least 50 %wt, at least 70 %wt, or at least 90 %wt of oxygen.
48. The system of any of aspects 40 to 43, wherein the gas in the gas source comprises at least 80 %wt of nitrogen, at least 85 %wt of nitrogen, at least 90 %wt of nitrogen or at least 95 %wt of nitrogen.
49. The system of any of aspects 40 to 44, further comprising a connection tube conducting the gas from the gas source to the plasma source, preferably wherein the connection tube conducts the gas from the gas source into the chamber through the gas inlet.
50. The system of any of the preceding aspects, further including a flow regulator.
51. The system of aspect 50, wherein the flow regulator is configured to provide for a gas volume flow of at least 0.1 standard liter per minute from the gas source.
52. The system of aspect 50, wherein the flow regulator is configured to provide for a gas volume flow of at least 0.3 standard liter per minute from the gas source.
53. The system of aspect 50, 51 or 52, wherein the flow regulator is configured to provide for a gas volume flow of 1.0 standard liter per minute or less from the gas source.
54. The system of aspect 50, 51 or 52, wherein the flow regulator is configured to provide for a gas volume flow of 0.8 standard liter per minute or less from the gas source.
55. The system of any of the preceding aspects, further comprising an exhalation tube for receiving exhaled air from the patient.
56. The system of aspect 55, wherein the exhaled air is led to a further plasma source of the system in order to at least partially inactivate one or more pathogens in the exhaled air.
57. The system of aspect 55 or 56, further comprising a filter for filtering one or more pathogens from the exhaled air, preferably wherein the filter is an air filter provided by a fibrous or porous material.
58. The system of aspect 57, wherein the further plasma source is located upstream of the filter.
59. The system of any of aspects 55 to 58, further comprising a filter for absorbing or adsorbing at least part of the reactive species within the exhaled air.
60. The system of any of the preceding aspects, wherein the system is a hand-held device.
61. The system of any of the preceding aspects, further comprising a mechanical ventilator, preferably a positive pressure ventilator, wherein the species directing member is at least partially formed by a ventilation tube for intubating the patient.
62. The system of aspect 61, wherein the plasma source is located inside a housing of the mechanical ventilator.
63. The system of aspect 61 or 62, wherein the mechanical ventilator comprises a bladder, wherein the reactive species generated by the plasma source are conducted into the bladder.
64. The system of aspect 61, wherein the plasma source is located outside a housing of the mechanical ventilator, wherein the plasma source is connected to the ventilation tube via a Y connector.
65. The system according to any of the preceding aspects, further comprising an expandable cuff configured to restrict airflow through the respiratory tract, preferably wherein the cuff is configured to be expanded at a position distal from the second end of the species directing member.
61. Use of the system according to any of the preceding aspects for plasma treatment of a viral, bacterial and/or fungal infection in the oral cavity and/or along the respiratory tract in a patient.
62. A method for plasma treatment of a viral, bacterial and/or fungal infection in a patient comprising introducing the species directing member (6) of the system according to any of the preceding aspects into the respiratory tract, in particular into the interior of the nose, the throat, trachea and/or lungs, and/or into the oral cavity.
63. The method according to aspect 62, wherein the species directing member (6) is inserted less than 1 cm or less than 0.5 cm into the nose and/or wherein the species directing member (6) is inserted less than 10 cm, preferably less than 5 cm into the pharyinx.
64. Plasma as obtainable by the system according to any of the preceding aspects for use in medicine.
65. Plasma as obtainable by the system according to any of the preceding aspects for use in the treatment and/or prevention of a viral, bacterial and/or fungal infection in a patient.
66. Plasma for use in the treatment and/or prevention of a viral, bacterial and/or fungal infection in a patient.
67. The use according to aspect 61, the method according to aspect 62 or 63, or the plasma for use according to aspect 64, 65 or 66, wherein said viral infection to be treated and/or prevented is a viral infectious disease selected from the group consisting of infections by coronaviruses, preferably SARS-CoV, SARS-CoV-2, MERS; influenza viruses, parainfluenza viruses, respiratory syncytial viruses (RSV), rhinoviruses, adenoviruses, noroviruses, enteroviruses, human metapneumoviruses, herpesviruses, preferably HSV-1, HSV-2, VZV, EBV, HCMV, HHV-6, HHV-7, HHV-8.
68. The use according to aspect 61 or 67, the method according to aspect 62, 63 or 67, or the plasma for use according to any aspects of 64 to 67, wherein said viral infection is the virus infection disease is the virus infection disease caused by the severe acute respiratory syndrome coronavirus (SARS-CoV)-2, SARS-CoV-1, or Middle East respiratory syndrome coronavirus (MERS-CoV), preferably the virus infection disease COVID-19 caused by the coronavirus SARS-CoV-2.
69. The use according to aspect 61, 67 or 68, the method according to aspect 62, 63, 67 or 68, or the plasma for use according to any aspects of 64 to 68, wherein the system is inserted into the respiratory tract, in particular into the interior of the nose, the throat, trachea and/or lungs, and/or into the oral cavity with reactive species being generated by the plasma source at least twice a day for at least 2 minutes.

## Claims

1. A system (2) for treating and/or preventing a viral, bacterial and/or fungal infection in the oral cavity and/or along the respiratory tract, in particular the interior of the nose, throat, trachea and/or lungs, of a patient by reactive species generated by plasma, the system (2) comprising:
a plasma source (4) generating reactive species in a gas, the plasma source (4) being configured to be located outside a body of the patient,
a species directing member (6) forming at least one duct for guiding at least a part of the reactive species generated by the plasma source (4) into the oral cavity and/or the respiratory tract.

2. The system of claim 1, wherein the duct (6) comprises a first end (61) through which at least part of the reactive species generated by the plasma source (4) enter the duct and a second, open end (10) through which at least part of said reactive species are released, wherein the first end (61) is configured to be located outside the patient's body and/or the second end (10) is configured to be located in the oral cavity and/or in the respiratory tract.

3. The system of claim 2, wherein the duct (6) is at least partly formed by an elongate tubular member (8), wherein the tubular member (8) has a length (1) of at least 1 cm, at least 2 cm, at least 3 cm, at least 5 cm, at least 10 cm, or at least 30 cm.

4. The system of any of the preceding claims, wherein the plasma source (4) comprises a first electrode (12), a second electrode (14) and a dielectric member (16), wherein at least a part of the dielectric member (16) is disposed between the first electrode (12) and the second electrode (14), wherein preferably the first electrode (12) is a laminar shaped high-voltage electrode and/or wherein the second electrode (14) is a structured ground electrode, for example a mesh or comb shaped electrode.

5. The system of any of the preceding claims, wherein the plasma source forms a duct (40) through which the gas flows, wherein a first electrode of the plasma source is a part of the duct (40) or constitutes the duct (40), preferably wherein a second electrode of the plasma source is arranged along a main axis (A) of the duct (40).

6. The system of any of the preceding claims, further comprising a chamber (24) disposed between the plasma source (4) and the species directing member (6), wherein the chamber (24) comprises a chamber outlet opening (26), and wherein a first end of the species directing member (6) is fluidly connected to the chamber outlet opening (26).

7. The system of claim 6, wherein the chamber (24) is formed in a chamber member (22), wherein the chamber member (22) is manually detachable from the plasma source (4) and/or manually removably connectable to the plasma source (4), preferably via a snap-fit and/or friction-fit connection.

8. The system of any of the preceding claims, further comprising a gas flow generator (34) configured to generate a gas flow through the species directing member (6) and/or a gas flow (32) into the chamber (24)

9. The system of any of claim 8, wherein the gas flow generator (34) is a fan.

10. The system of any of claim 8 or 9, wherein the gas flow generator (34) is positioned outside the chamber (24), preferably upstream of the chamber (24).

11. The system of any of the preceding claims, further comprising a gas source, preferably a source of pressurized gas.

12. The system of any of the preceding claims, wherein the system is a hand-held device.

13. Plasma, preferably plasma as obtainable by the system of any one of claims 1 to 12, for use in the treatment and/or prevention of a viral, bacterial and/or fungal infection in the oral cavity and/or along the respiratory tract.

14. The plasma for use of claim 13, wherein said viral infection is a viral infectious disease selected from the group consisting of infections by coronaviruses, preferably SARS-CoV, SARS-CoV-2, MERS); influenza viruses, parainfluenza viruses, respiratory syncytial viruses (RSV), rhinoviruses, adenoviruses, noroviruses, enteroviruses, human metapneumoviruses, herpesviruses, preferably HSV-1, HSV-2, VZV, EBV, HCMV, HHV-6, HHV-7, HHV-8.

15. The plasma for use of claim 13 or 14, wherein said viral infectious disease is the virus infection disease COVID-19 caused by the coronavirus SARS-CoV-2.
